(19) Europäisches Patentamt
European Patent Office
Office européen des brevets

(11) **EP 4 516 123 A1**

(12) **EUROPEAN PATENT APPLICATION**
published in accordance with Art. 153(4) EPC

(43) Date of publication:
**05.03.2025 Bulletin 2025/10**

(21) Application number: 23796543.9

(22) Date of filing: **28.04.2023**

(51) International Patent Classification (IPC):
*A24B 13/00* (2006.01)   *A61K 9/10* (2006.01)
*A61K 31/465* (2006.01)   *A61K 47/04* (2006.01)
*A61P 25/26* (2006.01)

(52) Cooperative Patent Classification (CPC):
A24B 13/00; A61K 9/10; A61K 31/465;
A61K 47/02; A61P 25/26

(86) International application number:
**PCT/JP2023/016863**

(87) International publication number:
**WO 2023/210811 (02.11.2023 Gazette 2023/44)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **28.04.2022 JP 2022074801**

(71) Applicant: **Japan Tobacco Inc.
Tokyo 105-6927 (JP)**

(72) Inventors:
• **MIYAUCHI, Masato
Tokyo 130-8603 (JP)**

• **FURUKOSHI, Masashi
Tokyo 130-8603 (JP)**
• **MUTO, Hiromichi
Tokyo 130-8603 (JP)**
• **KOBAYASHI, Kei
Tokyo 130-8603 (JP)**
• **SASAKI, Keisuke
Tokyo 130-8603 (JP)**

(74) Representative: **Hoffmann Eitle
Patent- und Rechtsanwälte PartmbB
Arabellastraße 30
81925 München (DE)**

(54) **ORAL COMPOSITION CONTAINING INORGANIC POROUS MATERIAL**

(57)    An oral composition comprising: an inorganic porous material having an average particle size of 60 $\mu$m or more, as a base material; and nicotine, wherein, in the inorganic porous material, the pore volume of pores having a pore size not less than 2 nm but less than 50 nm, as measured by a nitrogen adsorption method and calculated by the BJH method, is 0.50 cm$^3$/g or more.

**Description**

TECHNICAL FIELD

The present invention relates to an oral composition containing an inorganic porous material.

BACKGROUND ART

Oral compositions which are fillings of oral pouch products contain water. Therefore, stability may decrease due to, for example, agglomeration of the compositions during storage.
Incidentally, porous silica materials are used as, for example, carriers for pharmaceutical products by utilizing characteristics of their pores (refer to, PTL 1 and PTL 2).

CITATION LIST

PATENT LITERATURE

PTL 1: Japanese Unexamined Patent Application Publication (Translation of PCT Application) No. 2015-536939
PTL 2: Japanese Unexamined Patent Application Publication (Translation of PCT Application) No. 2006-518761

SUMMARY OF INVENTION

TECHNICAL PROBLEM

The above patent literatures do not disclose that porous silica materials are used in oral compositions containing nicotine, much less describe storage stability of the oral compositions. In view of such circumstances, an object of the present invention is to provide an oral composition and an oral pouch product that have good storage stability.

SOLUTION TO PROBLEM

The above problem is solved by the present invention described below.

Aspect 1

An oral composition containing:

an inorganic porous material having an average particle size of 60 $\mu$m or more, as a substrate; and
nicotine,
wherein, in the inorganic porous material, a pore volume of pores having a pore size of 2 nm or more and less than 50 nm, as measured by a nitrogen adsorption method and calculated by a BJH method, is 0.50 cm$^3$/g or more.

Aspect 2

The oral composition according to aspect 1,
wherein the inorganic porous material is selected from the group consisting of silica, silicate, alumina, zeolite, hydroxyapatite, hydrotalcite, and combinations thereof.

Aspect 3

The oral composition according to aspect 1 or 2,
wherein the inorganic porous material has a BET specific surface area of 200 to 1,200 m$^2$/g.

Aspect 4

The oral composition according to any one of aspects 1 to 3,

wherein V2/(V1 + V2 + V3) $\geq$ 35% by volume,
where V1 is a pore volume of pores having a pore size of less than 2 nm, as measured by the nitrogen adsorption method and calculated by an HK method,

V2 is a pore volume of pores having a pore size of 2 nm or more and less than 50 nm, as measured by the nitrogen adsorption method and calculated by the BJH method, and

V3 is a pore volume of pores having a pore size of 50 nm or more, as measured by a mercury intrusion method.

Aspect 5

The oral composition according to any one of aspects 1 to 4, having a water content of 10% by weight or more.

Aspect 6

The oral composition according to any one of aspects 1 to 5, wherein the oral composition contains the inorganic porous material in an amount of 6% by weight or more.

Aspect 7

An oral pouch product including:

the oral composition according to any one of aspects 1 to 6; and
a packaging material that packages the oral composition.

ADVANTAGEOUS EFFECTS OF INVENTION

The present invention can provide an oral composition and an oral pouch product that have good storage stability.

DESCRIPTION OF EMBODIMENTS

The present invention will be described in detail below. In the present invention, "X to Y" includes end values thereof, that is, X and Y. An oral product refers to a product whose active ingredient is ingested through the oral mucosa via saliva while the product is present in the oral cavity. An oral composition refers to a composition used in such an oral product.

1. Oral Composition

An oral composition according to the present embodiment (hereinafter, also simply referred to as a "composition") contains a specific inorganic porous material as a substrate, and nicotine. The substrate is a material that forms a matrix of the composition and is also referred to as an excipient.

(1) Inorganic Porous Material

The inorganic porous material has pores having a pore size of 2 nm or more and less than 50 nm (hereinafter, also referred to as "mesopores"), in which the pore volume of the pores is 0.50 $cm^3/g$ or more. The pore volume of the pores is calculated by applying a BJH method to a nitrogen adsorption isotherm obtained by measurement by a nitrogen adsorption method. Specifically, the pore volume (hereinafter, also referred to as "mesopore volume") can be determined by determining a nitrogen gas adsorption isotherm of the inorganic porous material, and accumulating a pore volume in a predetermined range of the pore size which is analyzed by applying the BJH method to the nitrogen gas adsorption isotherm. The nitrogen adsorption method can be performed in accordance with a known method; however, before the measurement, the inorganic porous material is preferably subjected to pretreatment. For silica, the pretreatment may be heating at 200°C to 250°C for two hours, and, for cellulose, the pretreatment may be heating at 105°C for one hour. The BJH method is an analysis method in which mesopores are assumed to have cylindrical shapes on the basis of the Kelvin's capillary condensation theory.

An inorganic porous material having a mesopore volume within the above range has good flowability. This is probably because water is adsorbed in mesopores to avoid agglomeration of the inorganic porous material or agglomeration of the inorganic porous material and another material, although the reason is not limited thereto. Accordingly, in an oral composition containing the inorganic porous material, uneven distribution of the composition does not occur during storage, and agglomerates are unlikely to be formed. Thus, the oral composition containing the inorganic porous material has good storage stability. From this viewpoint, the lower limit value of the mesopore volume is preferably 0.70 $cm^3/g$ or more. The upper limit value is not limited but is preferably 1.5 $cm^3/g$ or less.

The inorganic porous material preferably has a BET specific surface area of 200 to 1,200 m$^2$/g. A BET specific surface area within this range achieves the effect that liquid components, such as a flavoring agent and a sweetener, contained in the oral composition are efficiently adsorbed to maintain the taste of the product. From this viewpoint, the BET specific surface area is more preferably 200 to 500 m$^2$/g.

When a pore volume of micropores having a pore size of less than 2 nm, as measured by the nitrogen adsorption method and calculated by an HK method is represented by V1, a pore volume of mesopores having a pore size of 2 nm or more and less than 50 nm, as measured by the nitrogen adsorption method and calculated by the BJH method is represented by V2, and a pore volume of macropores having a pore size of 50 nm or more, as measured by a mercury intrusion method is represented by V3, a relationship below is preferably satisfied.

$$V2/(V1 + V2 + V3) \geq 35\%$$

by volume

When the volume fraction satisfies this range, the effect of improving the flowability becomes more noticeable. From this viewpoint, the volume fraction is more preferably 35% to 99% by volume.

The mercury intrusion method is a method in which mercury is intruded into pores in a solid surface, and the pore distribution and the pore volume are determined from the relationship between the pressure applied at that time and the volume of mercury intruded. The HK method is a method used for the measurement of micropores and is a method for determining a pore size distribution from calculations of interactions between adsorbed molecules and interactions between adsorbed molecules and pore wall atoms.

The inorganic porous material has an average particle size of 60 μm or more. The average particle size of each material is a particle size at a cumulative volume of 50% (D50) in a particle size distribution determined by laser diffraction particle size distribution measurement. The laser diffraction particle size distribution measurement can be performed using, for example, Mastersizer 3000 (manufactured by Malvern Panalytical Ltd.). The average particle size means a particle size at a cumulative volume of 50% (D50) in a particle size distribution, unless otherwise specified. If the average particle size is less than the lower limit value, flowability of the oral composition decreases. If the average particle size is excessively large, the user is likely to perceive the roughness due to particles of the inorganic porous material and may find the mouthfeel of the product unpleasant. From this viewpoint, the lower limit value of the average particle size is preferably 60 μm or more, more preferably 100 μm or more, and the upper limit value is preferably 500 μm or less, more preferably 300 μm or less.

The inorganic porous material is preferably selected from the group consisting of silica, silicate, alumina, zeolite, hydroxyapatite, hydrotalcite, and combinations thereof. Of these, silica is preferred from the viewpoints of, for example, availability and little influence on flavor.

In the composition, the inorganic porous material content is preferably 6% by weight or more. The content refers to an amount in an absolute dry state unless otherwise specified. The lower limit value of the content is preferably 10% by weight or more, more preferably 15% by weight or more. The upper limit of the content is not limited but is typically 70% by weight or less, preferably 68% by weight or less, more preferably 65% by weight or less from the viewpoint of the limit to which other raw materials can be blended.

(2) Nicotine

The composition contains nicotine. Nicotine may be contained in the form of nicotine alone or contained in the form of a nicotine-containing raw material. The nicotine-containing raw material refers to a raw material containing nicotine such as a nicotine salt or stabilized nicotine. An example of stabilized nicotine is a nicotine-carrying substance, such as nicotine supported on an ion-exchange resin. The ion-exchange resin may be a weakly acidic cation-exchange resin. The ion-exchange resin on which nicotine is supported can be specifically a resin complex that contains, for example, 10% by weight or more and 20% by weight or less of nicotine and is referred to as nicotine polacrilex. The ion-exchange resin used in nicotine polacrilex is a weakly acidic cation-exchange resin. When nicotine polacrilex is used, the amount of nicotine polacrilex added relative to the oral composition is typically, 0.5% by weight or more, preferably 10% by weight or more, more preferably 2.0% by weight or more. On the other hand, from the viewpoint of taste, the amount of nicotine polacrilex added relative to the composition is typically 15.0% by weight or less, preferably 12.0% by weight or less, more preferably 10.0% by weight or less.

The nicotine-containing raw material may be, for example, a tobacco material containing a tobacco powder obtained by grinding tobacco leaves. The tobacco powder may include, for example, shreds, a fine powder, or fibers of the lamina of dried tobacco leaves and is prepared by, for example, the method described later. Tobacco leaves may include mesophyll (lamina), leaf veins (stem), or roots. The tobacco material may basically include elements derived from midribs or roots of tobacco leaves, in addition to a tobacco powder obtained from the lamina of tobacco leaves.

The particle size of the tobacco powder is not limited. However, from the viewpoints of making fit in the oral cavity

EP 4 516 123 A1

satisfactory to enhance the feeling of use, and satisfactorily releasing a flavor component contained in the tobacco powder into the oral cavity, the tobacco powder is preferably a powder that has passed through a 1.2 mm mesh, more preferably a powder that has passed through a 1.0 mm mesh.

Examples of the tobacco varieties serving as a raw material of the tobacco powder include, but are not particularly limited to, the genus *Nicotiana,* such as the flue-cured tobacco and burley tobacco of *Nicotiana tabacum* and the Brazilian tobacco of *Nicotiana rustica.* For the tobacco material and tobacco leaves described later, the same varieties as these can be used.

The tobacco powder is preferably prepared as follows. First, a base is added to and mixed with a tobacco powder obtained by grinding tobacco leaves. The base to be added is, for example, potassium carbonate or sodium carbonate, and the base is preferably add as an aqueous solution. A pH adjuster such as sodium dihydrogenphosphate may be added, for example, for the stabilization of nicotine during the production of an oral pouch product. The pH of the mixture after the addition of the base is preferably adjusted to 8.0 to 9.0. The tobacco powder content in this mixture is preferably 60% to 90% by weight.

After the addition of the base, heating is performed, for example, for 0.5 to 3 hours, preferably 0.8 to 2 hours under conditions under which, for example, the mixture temperature is 65°C to 90°C, preferably, the mixture temperature is 70°C to 80°C. The sterilization of the tobacco powder is thereby performed. The heating can be performed by either one or both of heating by steam injection and heating with a jacket. The pH of the mixture after the heating is preferably 8.0 to 9.0, and the water content of the mixture after the heating is preferably 10% to 50% by weight.

In an embodiment, after the heating, the resulting processed tobacco powder may be subjected to drying treatment by performing heating with a jacket alone, after steam injection is stopped as necessary. Subsequently, the tobacco powder may be cooled at about 15°C to 25°C for about one hour.

When a tobacco material containing a tobacco powder is used, the amount of tobacco material added relative to the oral composition is typically 0.001% by weight or more, preferably 0.01% by weight or more, more preferably 0.05% by weight or more. On the other hand, from the viewpoint of taste, the amount of tobacco material added relative to the composition is typically 90% by weight or less, preferably 80% by weight or less, 70% by weight or less, 45% by weight or less, 40% by weight or less, or 30% weight or less.

The nicotine-containing raw material may be a nicotine-containing extraction liquid obtained through extraction from a nicotine-containing substance, such as tobacco leaves.

Of the embodiments described above, the use of a nicotine-carrying substance is preferred from the viewpoints of appropriate supply of nicotine and the ease of handling In addition, when a tobacco powder is added, the color of the oral composition and the oral product usually tends to be the color of tobacco leaves. In contrast, when a colorless nicotine-containing compound is used, a white composition and a white oral product can be provided. For users who like white oral products, such an embodiment is advantageous. The raw materials described above may be used alone or in combination of two or more thereof.

The total nicotine content in the composition is typically, but not limited to, 0.1% to 5.0% by weight from the viewpoint of user preference. The total nicotine content in the composition is typically, 0.1% by weight or more, preferably 10% by weight or more, more preferably 2.0% by weight or more. On the other hand, from the viewpoint of taste, the total nicotine content in the composition is typically 5.0% by weight or less, preferably 4.0% by weight or less, more preferably 3.0% by weight or less. Accordingly, when a nicotine-containing raw material is used as a vegetable alkaloid, the amount of the raw material is adjusted such that the total nicotine content is within this range. When nicotine is present as ions, the above content is a content as nicotine ions. The nicotine content in the composition can be measured by, for example, gas chromatography-mass spectrometer (GC-MS) or liquid chromatography (LC, UV detection).

(3) pH Adjuster

The composition contains a pH adjuster. The pH adjuster is not limited and is preferably one that is allowed to be added to food. Examples thereof include sodium carbonate, sodium hydrogencarbonate, potassium carbonate, potassium hydrogencarbonate, potassium phosphate, anhydrous sodium phosphate, sodium dihydrogenphosphate, and sodium citrate. Of these, sodium phosphate, potassium carbonate, or sodium dihydrogenphosphate is preferred from the viewpoints of influences on the taste of the product and product stability during storage. One of these pH adjusters may be used alone, or two or more of them may be used in combination in any ratio.

(4) Gelling Agent or Gelling Aid

The composition may contain a known gelling agent or the like. The gelling agent alleviates the uncomfortable feeling caused by a nonwoven fabric or the like when the product is taken into the oral cavity, in particular, at an initial point of time of taking it, and provides a favorable impression to the user. The gelling agent is preferably a polysaccharide having a carboxyl group as a functional group. For example, carrageenan, pectin, gum arabic, xanthan, gellan gum, and tragacanth gum are preferred. Furthermore, carrageenan, pectin, and gellan gum are preferred from the viewpoint that they are easily gelled in the presence of calcium ions, and that a carboxyl group and a cation can form a junction zone together to form a crosslinked structure. One of these may be used alone, or two or more of them may be used in combination in any ratio.

A gelling aid component is, for example, calcium ions, and examples of the source thereof (gelling aids) include, but are not limited to, halogen acid salts (such as chloride), citric acid, carbonate, sulfate, phosphate, and lactate of calcium. Of these, calcium lactate, calcium chloride, or calcium phosphate is preferred, and calcium lactate is particularly preferred, from the viewpoints of little influence on the taste, high solubility, and the pH after dissolution. One of these may be used alone or in combination of two or more thereof in any ratio.

Examples of the gelling aid component other than calcium ions include ions of metals such as magnesium, silver, zinc, copper, gold, and aluminum, with which the gelling agent can be bound through ionic bonds as with calcium ions, and ions of cationic polymers. Examples of the source thereof (other gelling aids) include halogen acid salts (such as chloride), citric acid, carbonates, sulfates, and phosphates of these metal ions and cationic polymers. One of these may be used alone, or two or more of them may be used in combination in any ratio.

(5) Release Agent

The composition may contain a known release agent. However, since the inorganic porous material, in particular, silica also has the function as a release agent, a release agent other than the inorganic porous material may not be contained.

(6) Water

The water content (moisture content) in the composition is 10% by weight or more in view of the ease of production of the composition. Furthermore, from the viewpoints of improving production efficiency of the composition, improving caking resistance of the composition, and reducing the stickiness of the composition, the lower limit of the water content is preferably 30% by weight or more, more preferably 45% by weight or more, and the upper limit is typically 60% by weight or less, preferably 50% by weight or less. The water content may be 40% by weight or less, may be 30% by weight or less, or may be 20% by weight or less. The water content can be controlled by adjusting the amount of water added or performing heating treatment or drying treatment during the production stage. The water content of the composition is controlled as appropriate according to the type of product (moist or dry). In the case of the moist type, for example, the water content is typically 20% to 60% by weight, preferably 30% to 50% by weight. On the other hand, in the case of the dry type, the water content is typically 5% to 20% by weight, preferably 10% to 15% by weight.

The water content (moisture content) of the composition can be measured with a heat-drying moisture meter (for example, HB 43-S: manufactured by METTLER TOLEDO). In the measurement, a sample is put in a predetermined container and heated to an attained temperature of 100°C. The measurement is terminated at the time when the amount of change becomes 1 mg or less per 60 seconds, and the moisture content is calculated from the weighed values before and after heating.

(7) Others

The composition may contain other substances besides the foregoing. Examples of the other substances include a flavoring agent, a sweetener, a humectant, a bitterness inhibitor, and an emulsifier. The contents of the substances are not limited, and their formulation can be appropriately adjusted according to the product design.

1) Flavoring Agent

The flavoring agent is not limited, and examples thereof include menthol, leaf tobacco extract, natural vegetable flavoring agents (e.g., cinnamon, sage, herb, chamomile, kudzu, sweet Hydrangea leaves, clove, lavender, cardamom, Eugenia caryophyllus, nutmeg, bergamot, geranium, honey essence, rose oil, lemon, orange, cassia bark, caraway, jasmine, ginger, coriander, vanilla extract, spearmint, peppermint, cassia, coffee, celery, cascarilla, sandalwood, cocoa, ylang-ylang, fennel, anise, licorice, Saint John's bread, plum extract, and peach extract), saccharides (e.g., glucose, fructose, isomerized sugar, caramel, honey, and molasses), cocoas (e.g., powder and extract), esters (e.g., isoamyl acetate, linalyl acetate, isoamyl propionate, and linalyl butyrate), ketones (e.g., menthone, ionone, damascenones, and ethyl maltol), alcohols (e.g., geraniol, linalool, anethole, and eugenol), aldehydes (e.g., vanillin, benzaldehyde, and anisaldehyde), lactones (e.g., $\gamma$-undecalactone and $\gamma$-nonalactone), animal flavoring agents (e.g., musk, ambergris, civet, and castoreum), and hydrocarbons (e.g., limonene and pinene). One of these flavoring agents may be used alone, or two or more of them may be used in combination in any ratio.

2) Sweetener

Examples of the sweetener include, but are not limited to, sugar alcohols such as xylitol, maltitol, and erythritol; and acesulfame potassium, sucralose, and aspartame. From the viewpoint of taste adjustment, sugar alcohols are preferred. One of these sweeteners may be used alone, or two or more of them may be used in combination in any ratio.

The type of sugar alcohol is not particularly limited, and examples thereof include xylitol, maltitol, erythritol, sorbitol, mannitol, and lactitol. Of these, maltitol is preferred from the viewpoint of imparting a good taste. One of these substances may be used alone, or two or more of them may be used in combination in any ratio.

The sugar alcohol content in the composition (when the composition contains two or more sugar alcohols, their total content) is not limited. However, from the viewpoint of taste adjustment, the sugar alcohol content is typically 1% by weight or more, preferably 5% by weight or more, more preferably 10% by weight or more. The upper limit is typically 80% by weight or less, preferably 70% by weight or less, more preferably 60% by weight or less.

3) Bitterness Inhibitor

The bitterness inhibitor is not limited, but an example thereof is soybean lecithin. Soybean lecithin is a phospholipid, and examples thereof include phosphatidylcholine, phosphatidylethanolamine, and phosphatidic acid. One of these bitterness inhibitors may be used alone, or two or more of them may be used in combination in any ratio.

4) Humectant

The humectant is not limited, but examples thereof include polyhydric alcohols, such as glycerine and propylene glycol. From the viewpoint of product storage stability, glycerine is preferred. One of these humectants may be used alone, or two or more of them may be used in combination in any ratio.

5) Emulsifier or Surfactant

The emulsifier is are not limited, but examples thereof include emulsifiers that are added to food. The emulsifier may be one or more selected from the group consisting of sucrose fatty acid esters, organic acid glycerine fatty acid esters, polyglycerine fatty acid esters, and lecithins. Examples of sucrose fatty acid esters include sucrose palmitate and sucrose stearate. Examples of organic acid glycerine fatty acid esters include succinic acid glycerine fatty acid esters and diacetyltartaric acid glycerine fatty acid esters. Examples of polyglycerine fatty acid esters include decaglycerine fatty acid esters. The emulsifier content in the composition is preferably an amount such that the total content including the above-described polyglycerine fatty acid ester falls within the above ranges described as the contents of the polyglycerine fatty acid esters.

The degree of polymerization of glycerine in a polyglycerine fatty acid ester is preferably 2 to 10. The polyglycerine fatty acid ester functions as an emulsifier. Thus, incorporating a polyglycerine fatty acid ester holds a state in which the components in the composition are homogeneously mixed and can stably keep the flavor component; therefore, a good flavor of the composition can be achieved. In addition, the polyglycerine fatty acid ester can impart a moderate viscosity to the composition to allow the components to be bound together, and thus can suppress dryness of the composition to make the feeling of use, flavor, and the like good. In addition, even in the case where the composition is of dry type, which has a low water content (moisture content), it is possible to reduce the scattering of the composition when the composition is packed in an exterior material such as a pouch. Thus, incorporating a polyglycerine fatty acid ester in the composition enables improvements in production efficiency in the production of the oral product, such as the operating efficiency and the yield.

Polyglycerine fatty acid esters are fatty acid esters of a dehydrated condensate of glycerine. The degree of polymerization of the glycerine is typically 2 or more and may be 3 or more, and is typically 10 or less and may be 8 or less.

Fatty acid ester groups (RCOO- groups) in a polyglycerine fatty acid ester are derived from a fatty acid. The fatty acid is not limited, and may be a saturated fatty acid or may be an unsaturated fatty acid. From the viewpoints of a good flavor and production efficiency, the number of carbon atoms in the fatty acid is typically 10 or more, preferably 12 or more, more preferably 14 or more, still more preferably 16 or more, and the number of carbon atoms is typically 30 or less, preferably 26 or less, more preferably 22 or less, still more preferably 20 or less. The fatty acid may have a substituent or may be unsubstituted.

The number of fatty acid ester groups that one molecule of the polyglycerine fatty acid ester has is not limited as long as the polyglycerine fatty acid ester has a structure with which it can exert a function as an emulsifier and can be appropriately selected according to the degree of polymerization of glycerine and the number of hydroxyl groups derived from glycerine. The structure with which the polyglycerine fatty acid ester can exert a function as an emulsifier is a structure having both a fatty acid moiety, which serves as a lipophilic group, and a polyhydric alcohol moiety, which serves as a hydrophilic group. Specifically, it is only necessary that the number of fatty acid ester groups per molecule of the polyglycerine fatty acid ester be typically 1 or more. It is only necessary that the number of hydroxyl groups derived from glycerine be 1 or more.

The degree of polymerization of glycerine and the type and number of fatty acid ester groups in the polyglycerine fatty acid ester can be any combination of those described above. More specifically, the alcohol component of the polyglycerine fatty acid ester may be diglycerine, triglycerine, tetraglycerine, pentaglycerine, hexaglycerine, heptaglycerine, octaglycerine, nonaglycerine, or decaglycerine. The acid component of the polyglycerine fatty acid ester may be a fatty acid such as lauric acid, myristic acid, palmitic acid, stearic acid, oleic acid, linoleic acid, or $\alpha$-linolenic acid. The polyglycerine fatty acid ester may be a monoester, a diester, a triester, a tetraester, a pentaester, or the like. One of these polyglycerine fatty acid esters may be used alone, or two or more of them may be used in combination in any ratio.

From the viewpoints of a good flavor and the workability during production, the polyglycerine fatty acid ester is preferably one or more selected from diglycerine monofatty acid esters and decaglycerine fatty acid esters. A diglycerine monofatty acid ester is preferably selected from the group consisting of diglycerine monolaurate, diglycerine monomyristate, diglycerine monopalmitate, diglycerine monostearate, and diglycerine monooleate, more preferably diglycerine monooleate. A decaglycerine fatty acid ester is preferably selected from the group consisting of decaglycerine laurates, decaglycerine myristates, decaglycerine palmitates, decaglycerine stearates, and decaglycerine oleates, more preferably selected from the group consisting of decaglycerine monolaurate, decaglycerine monomyristate, decaglycerine monopalmitate, decaglycerine monostearate, and decaglycerine monooleate.

The polyglycerine fatty acid ester content in the composition (when the composition contains two or more polyglycerine fatty acid esters, their total content) is not limited; however, from the viewpoints of obtaining a good flavor and improving production efficiency, the content is typically 0.1% by weight or more, preferably 0.2% by weight or more, more preferably 0.3% by weight or more, still more preferably 0.5% by weight or more. From the viewpoint of imparting a moderate viscosity to the composition, the polyglycerine fatty acid ester content is typically 20.0% by weight or less, preferably 15.0% by weight or less, more preferably 10.0% by weight or less, still more preferably 8.0% by weight or less.

The HLB value of the polyglycerine fatty acid ester is not limited. However, from the viewpoints of obtaining a good flavor and improving production efficiency, the HLB value is typically 6.0 or more, preferably 7.0 or more, and is typically 20.0 or less, preferably 18.0 or less, more preferably 16.0 or less.

(8) Properties of Composition

1) pH

The pH of the composition is not limited. However, in view of the influence on the taste, the pH is typically 7.0 or more, preferably 7.5 or higher, more preferably 8.0 or more, and is typically 10.0 or less, preferably 9.5 or less, more preferably 9.0 or less. The pH is a value measured at 25°C.

The pH of the composition at a measurement temperature of 25°C can be measured with a pH analyzer (for example, LAQUA F-72 with a flat ISFET pH electrode: manufactured by HORIBA, Ltd) by adding 20 mL of water relative to 2 g of the composition, shaking the mixture for 10 minutes, and analyzing the supernatant. The calibration of the device is preferably conducted by, for example, three-point calibration using a phthalate pH standard solution (pH 4.01), a phosphate pH standard equimolal solution (pH 6.86), and a tetraborate pH standard solution (pH 9.18) (all available from FUJIFILM Wako Pure Chemical Corporation).

2) Flowability

The oral composition according to the present embodiment has good flowability. The flowability is also an indicator of the viscosity, adhesion to production apparatuses, caking resistance, and the stickiness of the composition. The composition with good flowability has low adhesion to production apparatuses, good caking resistance, and low stickiness and has good handling properties. The flowability of the composition is evaluated through a relative comparison of a shear stress value at a normal stress of 5.0 kPa at a measurement temperature of 22°C. The normal stress of 5.0 kPa is a value assuming a pressure load applied to the composition under its own weight during, for example, the production, transport, or storage, as a condition under which the adhesion to production apparatuses, caking, and stickiness of the composition can occur. The shear stress is preferably 4.15 kPa or more, more preferably 4.20 kPa or more, still more preferably 4.25 kPa or more and is preferably 5.85 kPa or less, more preferably 5.80 kPa or less. The shear stress value of the composition measured at a normal stress of 5.0 kPa is preferably 1.5 to 6 kPa.

The above shear stress of the composition at a normal stress of 5.0 kPa can be measured using a rheometer. For example, when FT4 Powder Rheometer manufactured by Freeman Technology Ltd. is used as the rheometer, the measurement is conducted under the following measurement conditions.

Measurement mode: standard program (25 mm_shear_9 kPa)
Measurement temperature: 22°C
Measurement humidity: 60%RH
Measurement vessel: A cylindrical vessel with an inside diameter of 25 mm; volume, 10 ml
Normal load: 3 to 9 kPa

Measurement raw materials are each passed through a sieve (1.18 mm openings), the resulting fine and uniform particles are used as measurement samples, and the measurement is conducted in accordance with the procedure of the rheometer.

3) Adhesion of Composition

As described above, adhesion of the composition is represented using, as an indicator, a shear stress at a normal stress of 0 kPa at a measurement temperature of 22°C. The normal stress of 0 kPa is a numerical value assuming a state in which no pressure is applied other than the pressure applied when a pouch product is pressed in the thickness direction after a user puts the pouch product in the mouth and before saliva penetrates the pouch product, that is, other than this pressure applied in the thickness direction.

As with the above measurement of flowability, the shear stress of the composition is measured at normal stresses of 3 kPa, 4 kPa, 5 kPa, 6 kPa, and 7 kPa, and a graph is constructed by plotting normal stress on the horizontal axis and plotting shear stress on the vertical axis. Since shear stress changes linearly with respect to normal stress, fitting of the graph is performed, and the shear stress at a normal stress of 0 kPa is calculated from the results of the fitting. The conditions for the fitting are described below.

From the values of shear stresses at the normal stresses (3 kPa, 4 kPa, 5 kPa, 6 kPa, and 7 kPa), a first-order regression line is derived by calculation. The values of the slope and the Y-intercept of the regression line are calculated. The calculated value of the Y-intercept is determined as the shear stress at a normal stress of 0 kPa.

4) Flow Function

The Mohr's stress circle is fitted to the first-order line related to the fitting used for calculating the shear stress at a normal stress of 0 kPa in the above evaluation of adhesion to determine the major principal stress and the unconfined yield strength, and the ratio of the major principal stress to the unconfined yield strength (major principal stress/ unconfined yield strength) is calculated. Thus, a flow function can be evaluated.

A larger value of the flow function (FF) indicates higher flowability.

(9) Method for Producing Composition

The composition is produced by any method, but is preferably produced through a step of mixing a substrate, nicotine, and optionally the components described above. The mixing can be performed by putting all raw materials into a mixer and mixing the raw materials.

In a preferred production method, the substrate, nicotine, and optionally water and other substances (such as a sweetener, a flavoring agent, and a humectant) are first mixed together to prepare a first mixture. During this mixing, heating may be performed. The order of mixing of the raw materials is not limited; the raw materials may be put into the mixer and mixed together in any order or simultaneously, or solid raw materials may be homogeneously mixed, liquid raw materials may then be added to the mixture, and mixing may be further performed. In view of workability, the latter embodiment is preferred.

To the mixture obtained as described above, an aqueous solution containing a pH adjuster, a sweetener such as acesulfame potassium, a flavoring agent such as menthol, a bitterness inhibitor such as soybean lecithin, and a humectant such as glycerine can optionally be added (additive addition step). The above additives may be added in the form of solids or may be added in the form of aqueous solutions in which the additives are dissolved in water. When the additives are added in the form of aqueous solutions, the additives may be dissolved in advance in predetermined amounts of water and added so that the final water content of the oral product can be achieved.

2. Oral Pouch Product

The oral product is used by taking the product in the mouth. An oral pouch product refers to a product including a sealed water-insoluble packaging material (also referred to as a pouch) and a composition (also referred to as a main material or filling) contained in the packaging material, in which saliva permeates through the pouch and dissolves components in the composition contained in the pouch, and the components can subsequently pass through the pouch and be carried into the oral cavity.

(1) Pouch

The pouch is not limited and a known one can be used as long as the pouch can package the filling therein, is insoluble in water, and is permeable to liquids (such as water and saliva) and water-soluble components in the filling. An example of the material of the pouch is a cellulose-based nonwoven fabric, and a commercially available nonwoven fabric may be used. A pouch product can be produced by forming a sheet made of such a material into a bag shape, packing a filling into the bag, and sealing the bag by means of heat sealing or the like.

The basis weight of the sheet is not particularly limited and is typically 12 gsm (g/m$^2$) or more and 54 gsm or less, preferably 24 gsm or more and 30 gsm or less. The thickness of the sheet is not particularly limited and is typically 100 $\mu$m or more and 300 $\mu$m or less, preferably 175 $\mu$m or more and 215 $\mu$m or less.

At least one of the inner surface and outer surface of the pouch may be partially coated with a water-repellent material. A water-repellent fluororesin is suitable for the water-repellent material. Specifically, an example of a water-repellent fluororesin of this type is AsahiGuard (registered trademark) manufactured by AGC Inc. Water-repellent fluororesins are applied to packaging materials for fat-containing foods and products, such as confectionery, dairy products, ready-prepared foods, fast foods, and pet foods. Accordingly, water-repellent fluororesins of this type are safe even when applied to a pouch to be placed in the oral cavity. This water-repellent material is not limited to a fluororesin and may be a material having a water-repellent effect, such as a paraffin resin, a silicone resin, or an epoxy resin.

The pouch may contain optional components. Examples of such components include raw materials for adjusting flavor and taste, flavoring agents, additives, tobacco extracts, and coloring agents. These components may be incorporated in any form. For example, the components may be applied to or infiltrated into the surface of the pouch or, when the pouch is composed of fibers, the components may be incorporated into the fibers.

The appearance of the pouch is also not limited. The pouch may be nontransparent, translucent, or transparent. When the pouch is translucent or transparent, the filling can be seen through.

The size of the oral pouch product is not limited. As for the size of the product before use, the lower limit of the long side may be 25 mm or more, 28 mm or more, 35 mm or more, or 38 mm or more. The upper limit may be 40 mm or less. The lower limit of the short side may be 10 mm or more or 14 mm or more. The upper limit may be 20 mm or less or 18 mm or less. The ratio of the weight of the filling to the total weight of the oral pouch product is not limited, but is typically 80% by weight or more, preferably 85% by weight or more, more preferably 90% by weight or more. The ratio is typically 99% by weight or less, preferably 97% by weight or less, more preferably 95% by weight or less.

(2) Filling

The oral pouch product is filled with the composition as a filling. The amount of filling per oral pouch product is preferably 0.4 to 1.5 g.

(3) Method for Producing Oral Pouch Product

The oral pouch product can be produced by packaging the composition with an exterior material (packaging step). The packaging method is not limited, and a known method can be applied. For example, a known method can be employed in which the composition is put into a bag-shaped nonwoven fabric, followed by sealing. In the packaging step, desired water may be further added after sealing (water addition step). For example, when the water content of the final composition is 50% by weight and the water content in the packed composition is 15% by weight, water equivalent to the remaining 35% by weight is added.

Items of aspects will be described below.

Aspect 1

An oral composition containing:

an inorganic porous material having an average particle size of 60 $\mu$m or more, as a substrate; and
nicotine,
wherein, in the inorganic porous material, a pore volume of pores having a pore size of 2 nm or more and less than 50 nm, as measured by a nitrogen adsorption method and calculated by a BJH method, is 0.50 cm$^3$/g or more.

Aspect 2

The oral composition according to aspect 1,
wherein the inorganic porous material is selected from the group consisting of silica, silicate, alumina, zeolite, hydroxyapatite, hydrotalcite, and combinations thereof.

Aspect 3

The oral composition according to aspect 1 or 2,
wherein the inorganic porous material has a BET specific surface area of 200 to 1,200 $m^2/g$.

Aspect 4

The oral composition according to any one of aspects 1 to 3,

wherein V2/(V1 + V2 + V3) $\geq$ 35% by volume,
where V1 is a pore volume of pores having a pore size of less than 2 nm, as measured by the nitrogen adsorption method and calculated by an HK method,
V2 is a pore volume of pores having a pore size of 2 nm or more and less than 50 nm, as measured by the nitrogen adsorption method and calculated by the BJH method, and
V3 is a pore volume of pores having a pore size of 50 nm or more, as measured by a mercury intrusion method.

Aspect 5

The oral composition according to any one of aspects 1 to 4, having a water content of 10% by weight or more.

Aspect 6

The oral composition according to any one of aspects 1 to 5, wherein the oral composition contains the inorganic porous material in an amount of 6% by weight or more.

Aspect 7

An oral pouch product including:

the oral composition according to any one of aspects 1 to 6; and
a packaging material that packages the oral composition.

EXAMPLES

[Example 1] Production of oral composition

Silica (SIPERNAT 2200, manufactured by Evonik Industries) (100.0 g) serving as a substrate, 3.87 g of nicotine, 45.1 g of an aqueous solution of anhydrous sodium phosphate, 4.79 g of an aqueous solution of anhydrous citric acid, 14.03 g of an aqueous solution of sodium chloride, and 23.4 g of an aqueous solution of acesulfame K were added, and these were mixed until homogeneous to obtain a mixture A. The obtained mixture A was subjected to jacket heating (can wall temperature: 100°C). The mixture was then cooled for 30 minutes under the condition of an ambient temperature of 20°C to obtain a mixture B (moisture after cooling: 11%). To the mixture B after cooling, 67.7 g of an aqueous solution of tripotassium phosphate, 28.5 g of an aqueous solution of trisodium phosphate, 20 g of an aqueous solution of gellan gum, and other components were added to obtain 263 g of an oral composition (moisture content 41.2% by weight, pH 8.51).

[Example 2]

An oral composition was produced in the same manner as in Example 1 except that 36.0 g of powder cellulose (VITACEL L00, manufactured by J. Rettenmaier & Söhne) and 66.0 g of the silica were mixed and used as a substrate.

[Comparative Example 1]

An oral composition was produced in the same manner as in Example 1 except that 100.0 g of microcrystalline cellulose (VIVAPUR 200, manufactured by J. Rettenmaier & Söhne) was used as a substrate. Table 1 shows the compositions, and Table 2 shows physical properties of the substrates.

[Table 1]

|  |  | Example 1 | Example 2 | Comparative Example 1 |
|---|---|---|---|---|
| Amount blended (% by weight) | Nicotine | 1.37 | 1.37 | 1.37 |
| | Substrate (Total) | | 33.14 | |
| | - Cellulose in substrate | - | 11.60 | 33.14 |
| | - Silica in substrate | 33.14 | 21.54 | - |
| | Tobacco | 0.02 | 0.02 | 0.02 |
| | Citric acid | 1.52 | 1.52 | 1.52 |
| | $NaH_2PO_4$ | 4.23 | 4.23 | 4.23 |
| | $K_3PO_4$ | 11.44 | 11.44 | 11.44 |
| | $Na_3PO_4$ | 1.25 | 1.25 | 1.25 |
| | NaCl | 1.33 | 1.33 | 1.33 |
| | Acesulfame K | 1.66 | 1.66 | 1.66 |
| | Gellan gum | 0.05 | 0.05 | 0.05 |
| | Flavoring agent | 2.98 | 2.98 | 2.98 |
| | Water | 41.00 | 41.00 | 41.00 |
| | Total | 100 | 100 | 100 |
| Water content | | 40.08 | 41.20 | 41.12 |
| pH | | 8.46 | 8.51 | 8.50 |

[Table 2]

|  | Mesopore volume* $cm^3/g$ | BET specific surface area $m^2/g$ |
|---|---|---|
| Silica (D50 = 200 $\mu$m) | 0.78 | 224 |
| Powder cellulose (D50 = 120 $\mu$m) | 0.01 | ND |
| Microcrystalline cellulose (D50 = 250 $\mu$m) | 0.01 | 6 |
| * Measured by nitrogen adsorption method and calculated using BJH method | | |

The evaluation results of workability and flowability in the production of the compositions in the examples described above are described below. The compositions obtained in Examples exhibited good flowability. As described above, in compositions with high flowability, uneven distribution of the compositions does not occur during storage, and agglomerates are unlikely to be formed. The oral compositions of Examples have good storage stability.

Evaluation indicators related to the yield and adhesion to a machine were as described below.

A: Good (good yield, good powder properties)
B: Although it is inferior to A, it is possible to use in the later step of forming a pouch.
C: It is not possible to use in the later step of forming a pouch.

[Table 3]

|  |  | Example 1 | Example 2 | Comparative Example 1 |
|---|---|---|---|---|
| Evaluation indicator | Yield | A | A | C |
|  | Adhesion to machine | A | B | C |

[Table 4]

|  | Normal load (kPa) | Example 1 | Example 2 | Comparative Example 1 |
|---|---|---|---|---|
| Shear stress (kPa) | 7 | 4.3 | 6.4 | 9.6 |
|  | 6 | 3.7 | 5.6 | 9.0 |
|  | 5 | 3.1 | 5.0 | 8.2 |
|  | 4 | 2.5 | 4.3 | 7.4 |
|  | 3 | 1.9 | 3.5 | 6.4 |
| Flow Function |  | 25.0 | 3.5 | 1.5 |

Measurements were performed as described below.

<Average Particle Size>

The average particle size of each material means a particle size at a cumulative volume of 50% (D50) in a particle size distribution determined by laser diffraction particle size distribution measurement. The laser diffraction particle size distribution measurement was performed using Mastersizer 3000 (manufactured by Malvern Panalytical Ltd.).

<Amount of Water>

The measurement was conducted with a heat-drying moisture meter (HB 43-S: manufactured by METTLER TOLEDO). In the measurement, a sample was put in a predetermined container and heated to an attained temperature of 100°C. The measurement was terminated at the time when the amount of change became 1 mg or less per 60 seconds, and the moisture content was calculated from the weighed values before and after heating.

<Mesopore Analysis by Nitrogen Adsorption Method>

Pretreatment was conducted under the following conditions.

Silica: 250°C, heating for two hours
Cellulose: 105°C, heating for one hour

Subsequently, a nitrogen adsorption isotherm was obtained, and the mesopore volume was determined by the BJH method.
A gas adsorption amount measurement apparatus AutoSorb-1 (manufactured by Quantachrome Instruments) was used as the measurement apparatus.

<pH>

The measurement was conducted with a pH analyzer (LAQUA F-72 with a flat ISFET pH electrode: manufactured by HORIBA, Ltd.) by adding 20 mL of water relative to 2 g of a composition, shaking the mixture for 10 minutes, and analyzing the supernatant. The calibration of the device was conducted by, for example, three-point calibration using a phthalate pH standard solution (pH 4.01), a phosphate pH standard equimolal solution (pH 6.86), and a tetraborate pH standard solution (pH 9.18) (all available from FUJIFILM Wako Pure Chemical Corporation).

<Flowability>

(Shear Stress)

Shear stresses at normal stresses (normal loads) of 3 to 7 kPa at a measurement temperature of 22°C were measured using a rheometer (FT4 Powder Rheometer manufactured by Freeman Technology Ltd.). Measurement conditions were as follows.

Measurement mode: standard program (25 mm_shear_9 kPa)
Measurement temperature: 22°C
Measurement humidity: 60%RH
Measurement vessel: A cylindrical vessel with an inside diameter of 25 mm; volume, 10 ml
Normal load: 3 to 7 kPa

Measurement raw materials were each passed through a sieve (1.18 mm openings), the resulting fine and uniform particles were used as measurement samples, and the measurement was conducted in accordance with the procedure of the rheometer. The shear stress at a normal load of 5 kPa was used as an indicator of flowability.

(Flow Function)

Determination was conducted as described below.

1) A shear stress at a normal stresses of 0 kPa was first determined as described below.

A graph was constructed by plotting the normal stress on the horizontal axis and plotting the shear stress on the vertical axis. Since shear stress changes linearly with respect to normal stress, fitting of the graph was performed, and the shear stress at a normal stress of 0 kPa was calculated from the results of the fitting. The conditions for the fitting are described below.

From the values of shear stresses at the normal stresses (3 kPa, 4 kPa, 5 kPa, 6 kPa, and 7 kPa), a first-order regression line is derived by calculation. The values of the slope and the Y-intercept of the regression line were calculated. The calculated value of the Y-intercept was determined as the shear stress at a normal stress of 0 kPa.

2) The Mohr's stress circle was fitted to the first-order line to determine the major principal stress and the unconfined yield strength, and the ratio of the major principal stress to the unconfined yield strength (major principal stress/ unconfined yield strength) was calculated to determine a flow function.

A larger value of the flow function (FF) indicates higher flowability.

**Claims**

1. An oral composition comprising:

   an inorganic porous material having an average particle size of 60 $\mu$m or more, as a substrate; and
   nicotine,
   wherein, in the inorganic porous material, a pore volume of pores having a pore size of 2 nm or more and less than 50 nm, as measured by a nitrogen adsorption method and calculated by a BJH method, is 0.50 cm$^3$/g or more.

2. The oral composition according to claim 1,
   wherein the inorganic porous material is selected from the group consisting of silica, silicate, alumina, zeolite, hydroxyapatite, hydrotalcite, and combinations thereof.

3. The oral composition according to claim 1 or 2,
   wherein the inorganic porous material has a BET specific surface area of 200 to 1,200 m$^2$/g.

4. The oral composition according to any one of claims 1 to 3,

   wherein V2/(V1 + V2 + V3) $\geq$ 35% by volume,
   where V1 is a pore volume of pores having a pore size of less than 2 nm, as measured by the nitrogen adsorption method and calculated by an HK method,

V2 is a pore volume of pores having a pore size of 2 nm or more and less than 50 nm, as measured by the nitrogen adsorption method and calculated by the BJH method, and
V3 is a pore volume of pores having a pore size of 50 nm or more, as measured by a mercury intrusion method.

5. The oral composition according to any one of claims 1 to 4, having a water content of 10% by weight or more.

6. The oral composition according to any one of claims 1 to 5, wherein the oral composition contains the inorganic porous material in an amount of 6% by weight or more.

7. An oral pouch product comprising:

the oral composition according to any one of claims 1 to 6; and
a packaging material that packages the oral composition.

## INTERNATIONAL SEARCH REPORT

| International application No. |
|---|
| **PCT/JP2023/016863** |

### A. CLASSIFICATION OF SUBJECT MATTER

*A24B 13/00*(2006.01)i; *A61K 9/10*(2006.01)i; *A61K 31/465*(2006.01)i; *A61K 47/04*(2006.01)i; *A61P 25/26*(2006.01)i
FI:   A24B13/00; A61K9/10; A61K31/465; A61K47/04; A61P25/26

According to International Patent Classification (IPC) or to both national classification and IPC

### B. FIELDS SEARCHED

Minimum documentation searched (classification system followed by classification symbols)

A24B13/00; A61K9/10; A61K31/465; A61K47/04; A61P25/26

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | US 2019/0246686 A1 (ALTRIA CLIENT SERVICES LLC) 15 August 2019 (2019-08-15) paragraphs [0006]-[0025] | 1-7 |
| Y | JP 2014-237700 A (PHARMACEUTICAL PRODUCTIONS, INC.) 18 December 2014 (2014-12-18) paragraphs [0010], [0029] | 1-7 |
| Y | WO 2016/014454 A1 (PHARMACEUTICAL PRODUCTIONS, INC.) 28 January 2016 (2016-01-28) paragraphs [0012], [0015], [0019] | 1-7 |
| Y | JP 2011-529842 A (EVONIK DEGUSSA GMBH) 15 December 2011 (2011-12-15) paragraphs [0001]-[0002], [0079]-[0081] | 1-7 |
| Y | WO 2021/116866 A1 (NICOVENTURES TRADING LIMITED) 17 June 2021 (2021-06-17) specification, page 25, lines 3-10, page 31, line 3 to page 32, line 10 | 5, 7 |

☐ Further documents are listed in the continuation of Box C.     ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **12 June 2023** | **27 June 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**EP 4 516 123 A1**

**INTERNATIONAL SEARCH REPORT**
Information on patent family members

International application No.

**PCT/JP2023/016863**

| Patent document cited in search report | | | Publication date (day/month/year) | Patent family member(s) | | | Publication date (day/month/year) |
|---|---|---|---|---|---|---|---|
| US | 2019/0246686 | A1 | 15 August 2019 | (Family: none) | | | |
| JP | 2014-237700 | A | 18 December 2014 | EP paragraphs [0011], [0030] WO CN | 3056220 2008/127609 101677994 | A1 A1 A | |
| WO | 2016/014454 | A1 | 28 January 2016 | US | 2016/0015683 | A1 | |
| JP | 2011-529842 | A | 15 December 2011 | US paragraphs [0001]-[0002], [0099]-[0101] WO CN KR | 2011/0136919 2010/012638 102112396 10-2011-0052671 | A1 A1 A A | |
| WO | 2021/116866 | A1 | 17 June 2021 | US | 2021/0169123 | A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015536939 W

- JP 2006518761 W